# EUROPEAN PATENT SPECIFICATION

(11) **EP 4 159 117 B1**
(45) Date of publication and mention of the grant of the patent: **04.09.2024**
(21) Application number: 22193845.9
(22) Date of filing: 05.09.2022
(51) Int. Cl.: A61B 5/0205, A61B 5/113, A61B 5/00

(54) **REAL-TIME MONITORING DEVICE FOR HUMAN BODY**
ECHTZEITÜBERWACHUNGSVORRICHTUNG FÜR DEN MENSCHLICHEN KÖRPER
DISPOSITIF DE SURVEILLANCE EN TEMPS RÉEL DU CORPS HUMAIN

(30) Priority: 11.09.2021 US 202163243108 P; 22.08.2022 US 202217892121
(43) Date of publication of application: 05.04.2023
(73) Proprietor: SiriuXense Co., Ltd., East Dist. Hsinchu City 300 (TW)
(72) Inventor: TSAI, Fu-Ji, 220 New Taipei City (TW); HUANG, Ji-De, 300 Hsinchu City (TW); HUNG, Ju-Yu, 413 Taichung City (TW); KUO, Chen-I, 114 Taipei City (TW); LU, Chih-Chien, 334 Taoyuan City (TW)
(74) Representative: Kurig, Thomas

(56) References cited:
- CN-U- 204 440 601
- US-A- 5 492 129
- US-A1- 2005 195 085
- US-A1- 2017 156 594
- F. BELLONI ET AL: "A new digital stethoscope with environmental noise cancellation", MACMESE'10: PROCEEDINGS OF THE 12TH WSEAS INTERNATIONAL CONFERENCE ON MATHEMATICAL AND COMPUTATIONAL METHODS IN SCIENCE AND ENGINEERING, 3 November 2010 (2010-11-03), pages 169 - 174, XP093017944, ISSN: 1792-6114, Retrieved from the Internet <URL:http://www.wseas.us/e-library/conferences/2010/Faro/MACMESE/MACMESE-26.pdf>
- KLUM MICHAEL ET AL: "Wearable Multimodal Stethoscope Patch for Wireless Biosignal Acquisition and Long-Term Auscultation", 2019 41ST ANNUAL INTERNATIONAL CONFERENCE OF THE IEEE ENGINEERING IN MEDICINE AND BIOLOGY SOCIETY (EMBC), IEEE, 23 July 2019 (2019-07-23), pages 5781 - 5785, XP033625125, DOI: 10.1109/EMBC.2019.8857210

## Description

### BACKGROUND OF THE INVENTION

### 1. Field of the Invention

The present invention relates to the technology field of an electronic device configured for monitoring physical conditions and/or physiological parameters from a human body, and more particularly to a real-time monitoring device for human body.

### 2. Description of the Prior Art

There have been a variety of baby monitoring systems proposed. For example, U.S. patent No. 9,402,596B1 discloses a bowel sound analysis system, U.S. patent No. 8,094,013B1 discloses a baby monitoring system, U.S. patent No. 8,461,996B2 discloses an infant monitor, and U.S. patent publication No. 2005/0195085A1 discloses a wireless monitoring system of diaper wetness, motion, temperature and sound. On the other hand, prior art document D1 (CN204440601U) discloses an intelligent baby monitor system comprising a baby monitor smart box meant to contact a baby body surface and a monitor base station. The monitor smart box is battery-powered and comprises a temperature sensor, a heart rate or respiration sensor, an acoustic sensor, and a gyro sensor.

Prior art document D2 (US5492129A) discloses a noise-reducing stethoscope, and prior art document D3 discloses a new digital stethoscope with environmental noise cancellation, of which D3 is a journal paper written by F. BELLONI et. al, and is published on ADVANCES in MATHEMATICAL and COMPUTATIONAL METHODS (ISSN: 1792-6114). In addition, D4 discloses a wearable multimodal stethoscope patch for wireless biosignal acquisition and long-term auscultation, where D4 is written by Michael Klum et. al, and is published in 2019 41st Annual International Conference of the IEEE Engineering in Medicine and Biology Society (DOI: 10.1109/EMBC.2019.8857210). Moreover, D5 (US 2005/0195085 A1) discloses a wireless monitoring system of diaper wetness, motion, temperature and sound, and D6 (US 2017/0156594 A1) discloses a device to determine and predict physilogical states of individuals.

According to the disclosures of U.S. patent No. 9,402,596B1, the bowel sound analysis system is configured for merely determining a health condition of the intestinal tract of a baby by collecting and analyzing intestinal motility signals, and fails to simultaneously measure physiological parameters (e.g., heart rate and respiratory rate) and/or determine physical conditions of the baby. On the other hand, according to the disclosures of U.S. patent No. 8,094,013B1, the baby monitoring system is configured for measuring breath rate and determining body orientation of a child, and is not allowed for being used to monitor at least one body activity like excretion. Furthermore, according to the disclosures of U.S. patent No. 8,461,996B1, the infant monitor is configured for measuring movements of an infant's body, so as to monitor breathing, heartbeat, body temperature, and the like. However, the infant monitor still fails to monitor or determine at least one body activity (e.g., excretion) of the infant. According to prior art document D1, the intelligent baby monitor comprises a baby monitor intelligent box, a baby monitor base station, an Internet server and a carer end monitor, wherein an intelligent box temperature sensor, a heart rate sensor, a sound sensor, a gyroscope sensor, an intelligent box microprocessor, an intelligent box wireless transceiver and a lithium ion charging battery are arranged in the baby monitor intelligent box. The intelligent baby monitor allows a carer to know the body conditions at any time and take care of the baby, and incapable of discovery of diseases timely can be prevented. According to prior art document D2, the noise-reducing stethoscope comprises a body sound sensor, an ambient noise sensor, and a difference comparator, in which the body sound sensor is for placement on a body to detect internal body sounds, and the ambient noise sensor is used for detecting ambient noise. Thus, the difference comparator receives a first electrical signal and a second electrical signal from the body sound sensor and the ambient noise sensor, respectively, and is configured to cancel noise artifacts in the first electrical signal by comparing the first electrical signal with the second electrical signal. According to prior art document D3, the proposed system is based on an electronic stethoscope with enhanced performances in environmental noise reduction, achieved by means of two microphones whose signals are combined by an adaptive algorithm. A software tool able to reproduce, visualize and analyze cardiac sounds completes the system. Experimental results show significant improvements in noise reduction, when the adaptive algorithm is applied. According to prior art document D4, a multimodal, wearable biosignal acquisition patch including stethoscope auscultation, single-lead ECG, impedance pneumography, ambient noise recording and 9-axial actimetry is disclosed. The first and second heart tone are clearly visible in the PCG and showed physiologically plausible morphology and delay. The high signal quality of both the ECG and PCG signal as well as the exceptional low noise floor in both signals are worth noting. With the ECG being one of the most studied biosignals, a solid and reliable modality is available.

According to above descriptions, it is understood that there are still rooms for improvement in the conventional baby monitoring system. In view of this fact, inventors of the present application have made great efforts to make inventive research and eventually provided a real-time monitoring device for human body (infant's body).

### SUMMARY OF THE INVENTION

The invention is defined by the appended claims. The primary objective of the present invention is to disclose a real-time monitoring device for simultaneously measuring physiological parameters (e.g., heart rate and respiratory rate) and determining physical conditions of a human body like baby's. The real-time monitoring device comprises a sensor module and a processor module, wherein the sensor module is adopted for contacting a body of a baby, so as to measure a body temperature from the body, collect a sound emitted from the body, and monitor a movement and/or a vibration of the body, thereby generating a body temperature sensing signal, a first sound signal and a body activity sensing signal. On the other hand, the processor module is coupled to the sensor module, and is configured for generating a second sound signal after collecting the sound emitted from the body and an ambient sound, and then determining whether the baby has a physical condition after applying a signal analyzing process to the body temperature sensing signal, the first sound signal, the second sound signal, and the body activity sensing signal. The physical condition includes: excretion, abnormal heart rate (HR), abnormal respiration rate (RR), emission of abnormal bowel sounds, airway obstruction, going into a deep sleep, going into a light sleep, and going into a paradoxical sleep. Moreover, after processing and analyzing the first sound signal, the second sound signal and the body activity sensing signal, the processor module also estimates physiological parameters of the baby, including heart rate and respiration rate.

For achieving the primary objective mentioned above, the present invention provides an embodiment of the real-time monitoring device for human body, comprising:
a sensor module, comprising a first body and a first circuit assembly disposed in the first body, wherein the first circuit assembly comprises a first microphone, a temperature sensor and an inertial sensor; and
a processor module, comprising a second body and a second circuit assembly disposed in the second body, wherein the second circuit assembly comprises a second microphone, a microprocessor, a memory, and a wireless transmission interface;
wherein the first body is allowed to be contacted a human body by a body contacting surface thereof, and the memory storing an application program including instructions, such that in case the application program is executed, the microprocessor being configured for:
   controlling the temperature sensor to measure a body temperature from the human body, thereby generating a body temperature sensing signal;
   controlling the first microphone to collect a sound emitted from the human body, thereby generating a first sound signal;
   controlling the inertial sensor to monitor a movement and/or a vibration of the human body, thereby generating a body activity sensing signal;
   controlling the second microphone to collect said sound emitted from the human body and an ambient sound, thereby generating a second sound signal;
   judging whether the human body has at least one physical condition by comparing the first sound signal with the second sound signal; and
   analyzing the body temperature sensing signal, the first sound signal, the second sound signal, and the body activity sensing signal, so as to determine said physical condition includes at least one selected from a group consisting of excretion, abnormal heart rate (HR), abnormal respiration rate (RR), emission of abnormal bowel sounds, airway obstruction, going into a deep sleep, going into a light sleep, and going into a paradoxical sleep.

In one embodiment, the application program consists of a plurality of subprograms, and the plurality of subprograms comprising:
a first subprogram, being compiled to be integrated in the application program by one type of programming language, and including instructions for configuring the microprocessor to control the temperature sensor to measure the body temperature from the human body;
a second subprogram, being compiled to be integrated in the application program by one type of programming language, and including instructions for configuring the microprocessor to control the first microphone and the second microphone to collect the sound emitted from the human body;
a third subprogram, being compiled to be integrated in the application program by one type of programming language, and including instructions for configuring the microprocessor to control the inertial sensor to monitor the movement and/or the vibration of the human body;
a fourth subprogram, being compiled to be integrated in the application program by one type of programming language, and including instructions for configuring the microprocessor to process the body temperature sensing signal, the first sound signal, the second sound signal, and/or the body activity sensing signal;
a fifth subprogram, being compiled to be integrated in the application program by one type of programming language, and including instructions for configuring the microprocessor to apply a signal synchronizing process to the body temperature sensing signal, the first sound signal, the second sound signal, and the body activity sensing signal according to four timestamps that are respectively contained in the body temperature sensing signal, the first sound signal, the second sound signal, and the body activity sensing signal; and
a sixth, being compiled to be integrated in the application program by one type of programming language, and including instructions for configuring the microprocessor to judge whether the human body has at least one physical condition and then determine said physical condition.

In one embodiment, the plurality of subprograms further comprises:
a seventh subprogram, being compiled to be integrated in the application program by one type of programming language, and including instructions for configuring the microprocessor to calculate an estimated body temperature according to the body temperature sensing signal, and to estimate at least one physiological parameter of the human body by processing the first sound signal, the second sound signal and the body activity sensing signal; wherein the physiological parameter is selected from a group consisting of heart rate (HR) and respiration rate (RR).

In one embodiment, the plurality of subprograms further comprises:
an eighth subprogram, being compiled to be integrated in the application program by one type of programming language, and including instructions for configuring the microprocessor to judge whether there is a well contact between the first body and the human body by analyzing the body temperature sensing signal, the body activity sensing signal, a first frequency band and a second frequency band of the first sound signal.

In one embodiment, the plurality of subprograms further comprises:
a ninth subprogram, being compiled to be integrated in the application program by one type of programming language, and including instructions for configuring the microprocessor to generate a warning signal in case of there is existing said physical condition and/or at least one said physiological parameter exceeding a normal range, and then to transmit the warning signal to an electronic device through the wireless transmission interface.

In one embodiment, the electronic device is selected from a group consisting of signal transceiver device, tablet computer, cloud server, laptop computer, desktop computer, all-in-one computer, smart phone, smart watch, and smart glasses.

In one embodiment, the memory is selected from a group consisting of embedded flash (eFlash) memory, flash memory chip, hard drive (HD), solid state drive (SSD), and USB flash drive.

In one embodiment, the microprocessor is provided with an analog-to-digital (A/D) convertor therein, and the A/D convertor directly digitizes the first sound signal, digitizes the second sound signal using a first sampling rate, and digitizes the body activity sensing signal using a second sampling rate.

In one embodiment, the first sampling rate is not greater than 4 KHz, and the second sampling rate is not greater than 120 Hz.

According to the claimed invention, the first body has a first accommodation space for receiving the first circuit assembly therein, and a first cover is connected to a first opening of the first accommodation space so as to shield the first circuit assembly.

According to the claimed invention, an aperture is formed on a bottom of the first accommodation space, such that the first microphone is exposed out of the first body via the aperture.

According to the claimed invention, a circular recess is formed on the body contacting surface of the first body, and the circular recess has a depth and a diameter in a range between 4.5 mm and 20 mm, such that a ratio of the diameter to the depth is not greater than 6.

In one embodiment, a minimum value of the depth is 1.5 mm.

In one embodiment, the second body has a second accommodation space for receiving the second circuit assembly therein, and a second cover is connected to a second opening of the second accommodation space so as to shield the second circuit assembly.

In one embodiment, a body connecting member is connected between the first body and the second body, and the body connecting member is provided with an electrical connecting component therein, such that the first circuit assembly is coupled to the second circuit assembly through the electrical connecting component.

In one embodiment, the real-time monitoring device according to the present invention further comprises:
an article supporting unit, being disposed in the second accommodation space, and consisting of a platform and a plurality of supporting rods; wherein the platform is faced to a bottom of the second accommodation space, and the second circuit assembly being positioned in a space formed by the plurality of supporting rods and a bottom surface of the platform.

In one embodiment, the processor module further comprises:
a wireless charging module, being disposed on a top surface of the platform, and being coupled to the second circuit assembly; and
a battery, being coupled to the second circuit assembly.

In one practicable embodiment, the second body, the body connecting member and the first body are allowed to be fixed on a mounting kit, such that after disposing the mounting kit on an article that is worn on the human body, the first body being set to contact the human body by the body contacting surface thereof. Moreover, in case of the first body being set to contact the human body, a device fixing member is allowed to be used in further fixing the second body on the article.

In another one practicable embodiment, the second body and the first body are allowed to be connected with a device fixing member, such that the second body and the first body are allowed to be attached onto the human body through the device fixing member, thereby making the first body 11 contact the human body by the body contacting surface thereof.

### BRIEF DESCRIPTION OF THE DRAWINGS

The invention as well as a preferred mode of use and advantages thereof will be best understood by referring to the following detailed description of an illustrative embodiment in conjunction with the accompanying drawings, wherein:
- FIG. 1A: shows a first stereo diagram of a real-time monitoring device for human body according to the present invention;
- FIG. 1B: shows a second stereo diagram of the real-time monitoring device;
- FIG. 2: shows a diagram for describing an application of the real-time monitoring device;
- FIG. 3A: shows a third stereo diagram of the real-time monitoring device;
- FIG. 3B: shows a fourth stereo diagram of the real-time monitoring device;
- FIG. 3C: shows a fifth diagram of the real-time monitoring device;
- FIG. 3D: shows a sixth stereo diagram of the real-time monitoring device;
- FIG. 4A: shows a seventh stereo diagram of the real-time monitoring device;
- FIG. 4B: shows an eighth stereo diagram of the real-time monitoring device;
- FIG. 5A: shows a first exploded diagram of the real-time monitoring device;
- FIG. 5B: shows a second exploded diagram of the real-time monitoring device;
- FIG. 6: shows a block diagram of a first microphone, a temperature sensor, an inertial sensor, a second microphone, a microprocessor, a memory, and a wireless transmission interface;
- FIG. 7: shows a measured data graph of the body activity sensing signal, the body temperature sensing signal and the first sound signal;
- FIG. 8: shows a FFT spectrogram of the first sound signal containing airway obstruction feature;
- FIG. 9: shows a measured data graph of the first sound signal, the FFT spectrogram of the first sound signal, the second sound signal, the FFT spectrogram of the second sound signal, and body activity sensing signal;
- FIG. 10: shows a measured data graph of the first sound signal, the FFT spectrogram of the first sound signal, the second sound signal, the FFT spectrogram of the second sound signal, and body activity sensing signal; and
- FIG. 11: shows a measured data graph of hearth rate signal and respiration rate signal.

### DETAILED DESCRIPTION OF THE PREFERRED EMBODIMENTS

To more clearly describe a real-time monitoring device for human body according to the present invention, embodiments of the present invention will be described in detail with reference to the attached drawings hereinafter.

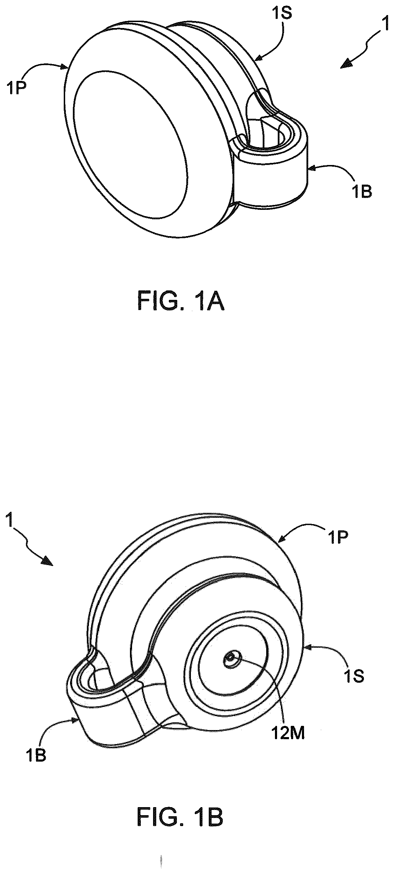

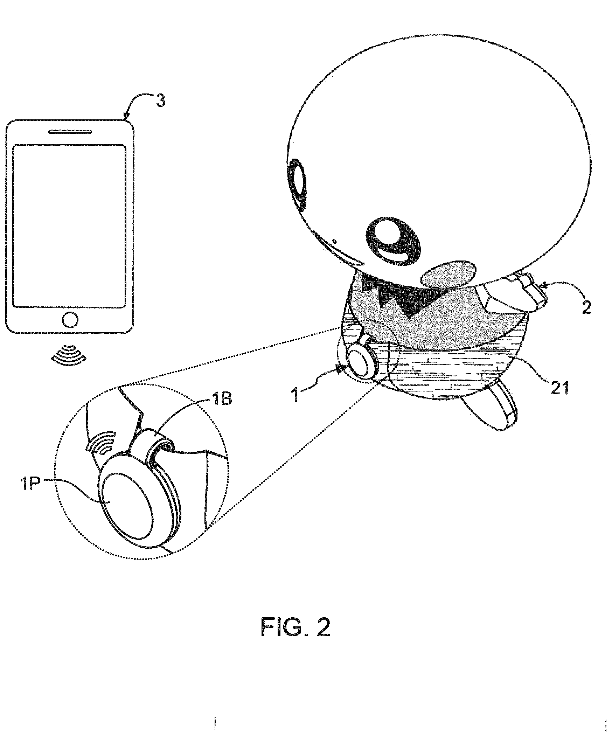

With reference to FIG. 1A and FIG. 1B, there are provided a first stereo diagram and a second stereo diagram of a real-time monitoring device for human body according to the present invention. The real-time monitoring device 1 is particularly designed for simultaneously measuring physiological parameters (e.g., heart rate and respiratory rate) and determining physical conditions of a human body like baby's. As FIG. 1A and FIG. 1B show, the real-time monitoring device 1 comprises a sensor module 1S, a processor module 1P and a body connecting member 1B connected between the sensor module 1S and the processor module 1P. In addition, FIG. 2 shows a diagram for describing an application of the real-time monitoring device. According to FIG. 1A, FIG. 1B and FIG. 2, it is understood that, the sensor module 1S, the processor module 1P and the body connecting member 1B are allowed to be fixed on a mounting kit 1K, such that the body connecting member 1B is bent to have a first curvature.

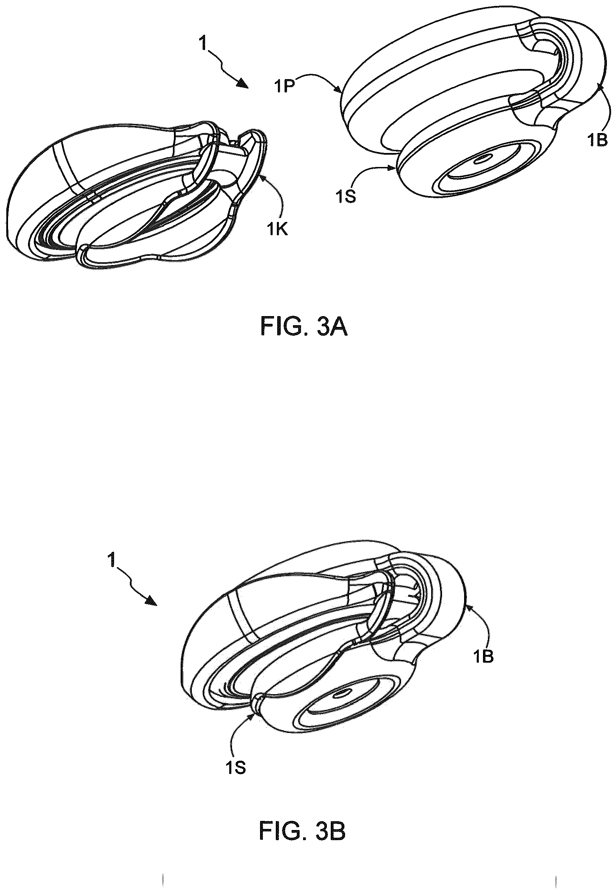

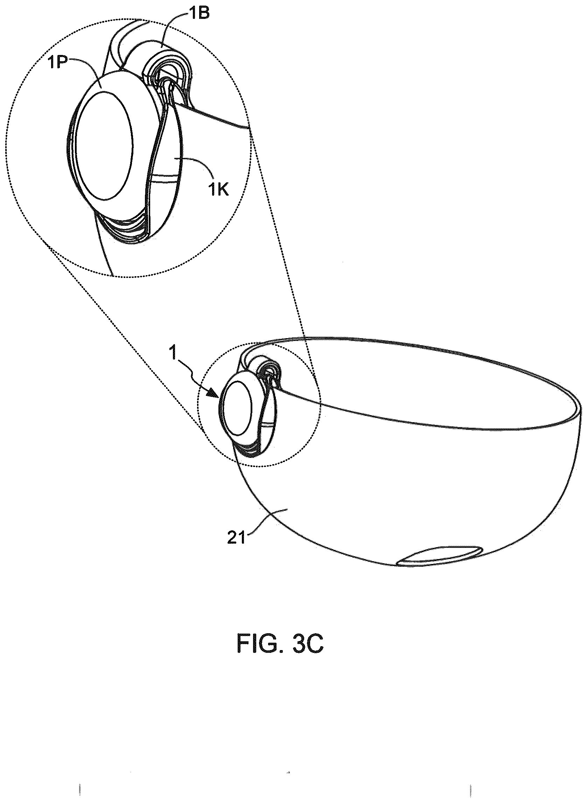

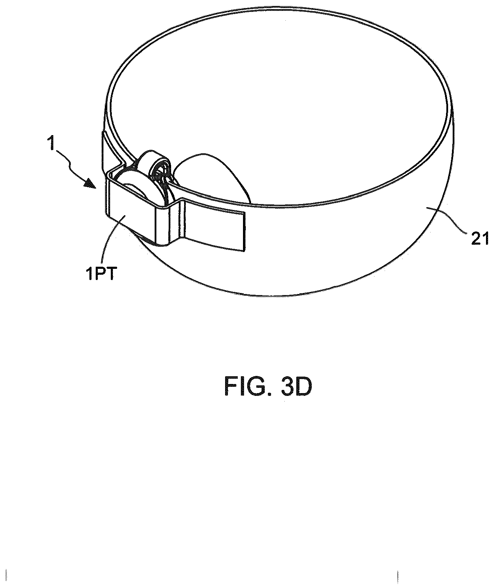

On the other hand, FIG. 3A, FIG.3B and FIG. 3C show a third stereo diagram, a fourth stereo diagram and a fifth stereo diagram of the real-time monitoring device, respectively. When using this real-time monitoring device 1, the second body 13, the body connecting member 1B and the first body 11 are allowed to be fixed on the mounting kit 1K, such that it is able to next dispose the mounting kit 1K on an article that is worn on the human body, e.g., a diaper 21 worn on a baby 2. In such case, the real-time monitoring device 1 is hung on the top opening edge of the diaper 21 via the mounting kit 1K, such that the sensor module 1S is set to contact the human body (e.g., a body 2 of a baby) by a body contacting surface thereof. Furthermore, FIG. 3D illustrates a sixth stereo diagram of the real-time monitoring device. As FIG. 3D shows, in case of the sensor module 1S being set to contact the body 2, a first device fixing member 1PT is allowed to be used in further fixing the processor module 1P on an article of the body 2 (i.e., diaper 21).

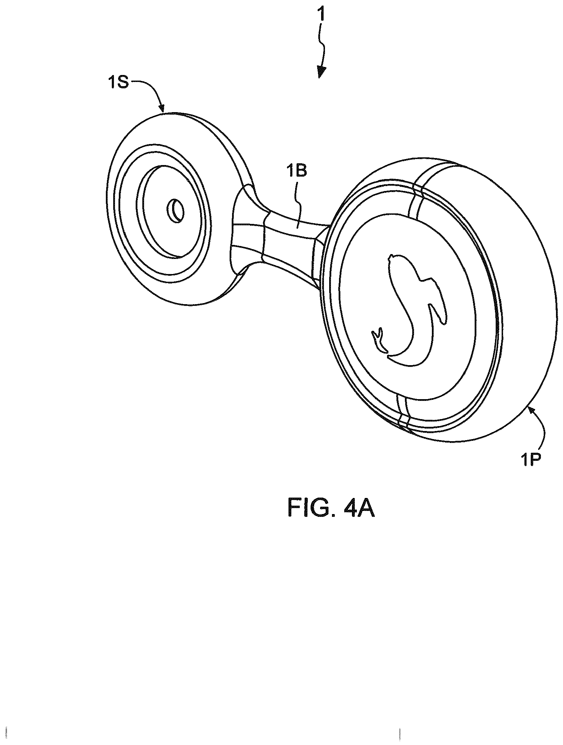

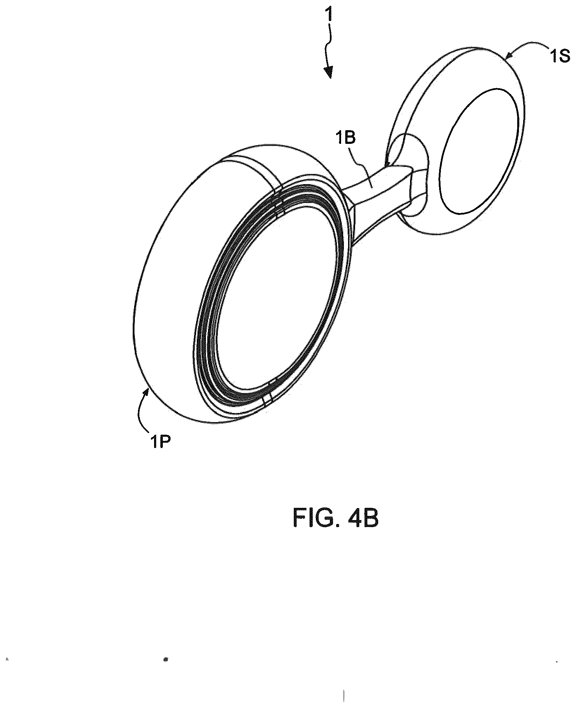

FIG. 4A and FIG. 4B illustrate a seventh stereo diagram and an eighth stereo diagram of the real-time monitoring device, respectively. In another practicable application, as FIG. 4A and FIG. 4B show, the real-time monitoring device 1 is allowed to be spread out, so as to make the body connecting member 1B has a second curvature smaller than the foregoing first curvature. In such case, the real-time monitoring device 1 is allowed to be connected with a second device fixing member (not shown), and then be attached onto the body 2 through the second device fixing member, thereby making the sensor module 1S contact the body 2 by the body contacting surface thereof.

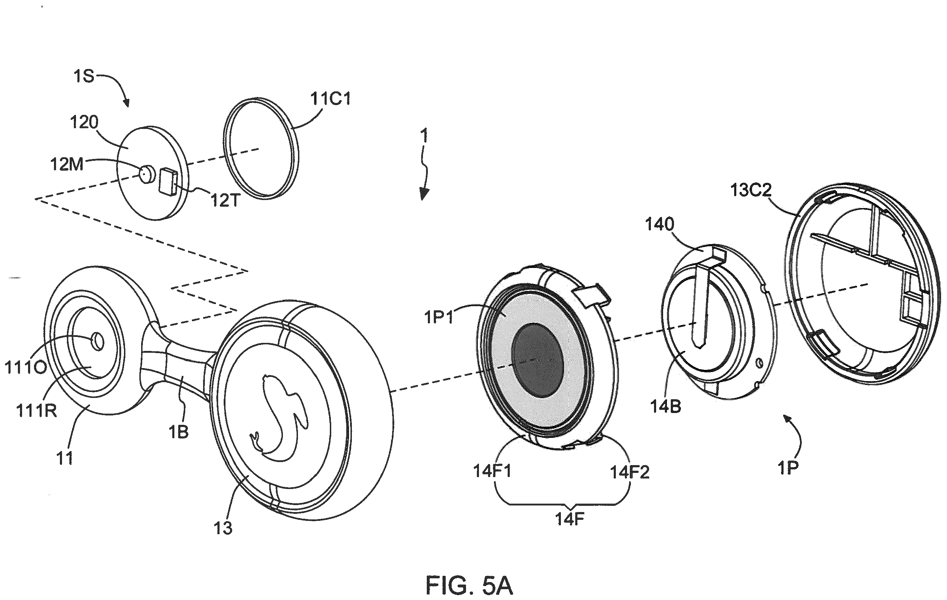

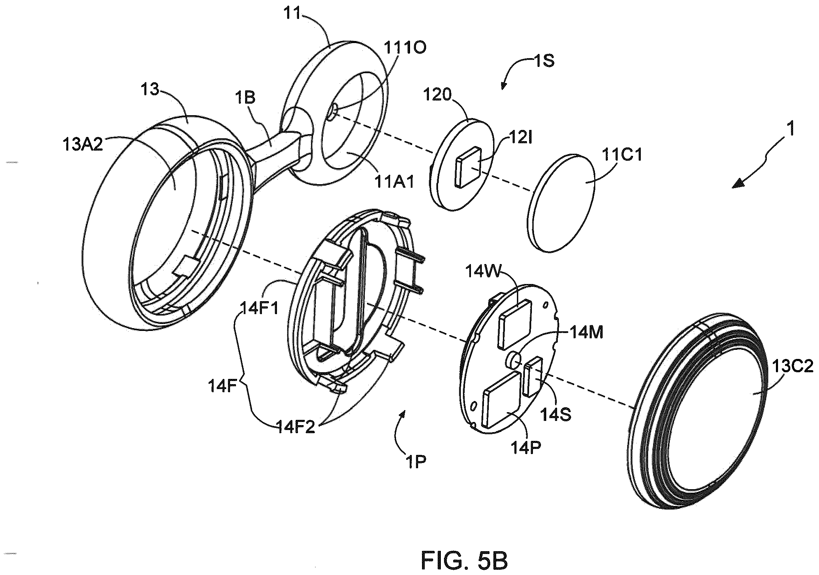

FIG. 5A and FIG. 5B illustrate a first exploded diagram and a second exploded diagram of the real-time monitoring device, respectively. As FIG. 5A and FIG. 5B show, the sensor module 1S comprises a first body 11 and a first circuit assembly disposed in the first body 11, of which the first circuit assembly comprises a first circuit board 120 and a first microphone 12M, a temperature sensor 12T and an inertial sensor 12I dispose on the first circuit board 120. On the other hand, the processor module 1P comprises a second body 13 and a second circuit assembly disposed in the second body 13, of which the second circuit assembly comprises a second circuit board 140 and a second microphone 14M, a microprocessor 14P, a memory 14S, and a wireless transmission interface 14W disposed on the second circuit board 140.

As described in more detail below, the first body 11 has a first accommodation space 11A1 for receiving the first circuit assembly therein, and a first cover 11C1 is connected to a first opening of the first accommodation space 11A1 so as to shield the first circuit assembly. Moreover, an aperture 111O is formed on a bottom of the first accommodation space 11A1, such that the first microphone 12M is exposed out of the first body 11 via the aperture 111O. On the other hand, the second body 13 has a second accommodation space 13A2 for receiving the second circuit assembly therein, and a second cover 13C2 is connected to a second opening of the second accommodation space 13A2 so as to shield the second circuit assembly. Particularly, the body connecting member 1B is connected between the first body 11 and the second body 13, and the body connecting member 1B is provided with an electrical connecting component therein, such that the first circuit assembly is coupled to the second circuit assembly through the electrical connecting component.

According to the present invention, a circular recess 111R is formed on the body contacting surface of the first body 11, and the circular recess 111R has a depth and a diameter in a range between 4.5 mm and 20 mm, such that a ratio of the diameter to the depth being not greater than 6. By such design, after the first body 11 is set to contact the human body (e.g., the baby's belly) by a body contacting surface thereof, the circular recess 111R helps the body contacting surface to well contact the skin of the baby's belly with high air tightness, thereby making an acoustic coupling path be formed between the first body 11 and a sound source portion of the baby (e.g., peritoneal cavity). It is worth further explaining that the human body is a low frequency resonator. Therefore, in case of there being a sound emitted by heart, lungs, respiratory tract, intestines, and/or excretion (i.e., the sound source portion), magnitude of the low frequency band of the sound would be amplified by the low frequency resonator, wherein said low frequency band includes sound signal falls below 25 Hz. Moreover, because there is an acoustic coupling path formed between the first body 11 and the sound source portion of the human body, the sound emitted by the human body is directly corrected by the first microphone 12M through the acoustic coupling path.

In a specific embodiment, the depth can be designed to have a minimum value of 1.5 mm. On the other hand, in case of the first body 11 being set to contact the human body by the body contacting surface thereof, the circular recess 111R is also allowed to prevent the aperture 111O (i.e., sound collecting hole for the first microphone 12M) from being plugged by the baby's belly. As described in more detail below, an article supporting unit 14F is disposed in the second accommodation space 13A2. As FIG. 5A and FIG. 5B show, the article supporting unit 14F consists of a platform 14F1 and a plurality of supporting rods 14F2, of which the platform 14F1 is faced to a bottom of the second accommodation space 13A2, and the second circuit assembly is positioned in a space formed by the plurality of supporting rods 14F2 and a bottom surface of the platform 14F1. Moreover, in the second body 13, there is a wireless charging module 1P1 disposed on a top surface of the platform 14F1 so as to be coupled to the second circuit assembly, and a battery 14B is coupled to the second circuit assembly. By such arrangements, it is allowed to put the real-time monitoring device 1 on a particularly-designed signal transceiver device, so as to make the second body 13 contact the signal transceiver device by a device contacting surface thereof. In such case, the signal transceiver device transmits electricity energy to the wireless charging module 1P1 through electromagnetic induction, such that the battery 14B is charged by the electricity energy.

As FIG. 1A, FIG. 1B, FIG. 2, FIG. 5A, and FIG. 5B show, the sensor module 1S is configured for measure a body temperature from the body 2, collect a sound emitted from the body 2, and monitor a movement and/or a vibration of the body 2, thereby generating a body temperature sensing signal, a first sound signal and a body activity sensing signal. On the other hand, the processor module 1P is coupled to the sensor module 1S, and is configured for generating a second sound signal after collecting the sound emitted from the body 2 and an ambient sound. As a result, after applying a signal analyzing process to the body temperature sensing signal, the first sound signal, the second sound signal, and the body activity sensing signal, the processor module 1P determines whether the body 2 has a physical condition or not. The physical condition includes: excretion, abnormal heart rate (HR), abnormal respiration rate (RR), emission of abnormal bowel sounds, airway obstruction, going into a deep sleep, going into a light sleep, and going into a paradoxical sleep. Moreover, after processing and analyzing the first sound signal, the second sound signal and the body activity sensing signal, the processor module 1P also estimates physiological parameters of the baby, including heart rate (HR) and respiration rate (RR). Of course, the processor module 1P can also calculate an estimated body temperature according to the body temperature sensing signal.

According to the present invention, the processor module 1P is further configured for generating a warning signal in case of there is existing said physical condition and/or at least one said physiological parameter exceeding a normal range, and then transmitting the warning signal to an electronic device 3 like the foregoing signal transceiver device through the wireless transmission interface 14W. Besides the signal transceiver device, the electronic device 3 can be a cloud server, a local server belong to a hospital, a postpartum center or an infant care center, and can also be a personal electronic device belong to the baby's parent, wherein the personal electronic device can be a tablet computer, a laptop computer, a desktop computer, an all-in-one computer, a smart phone, a smart watch, or a smart glasses.

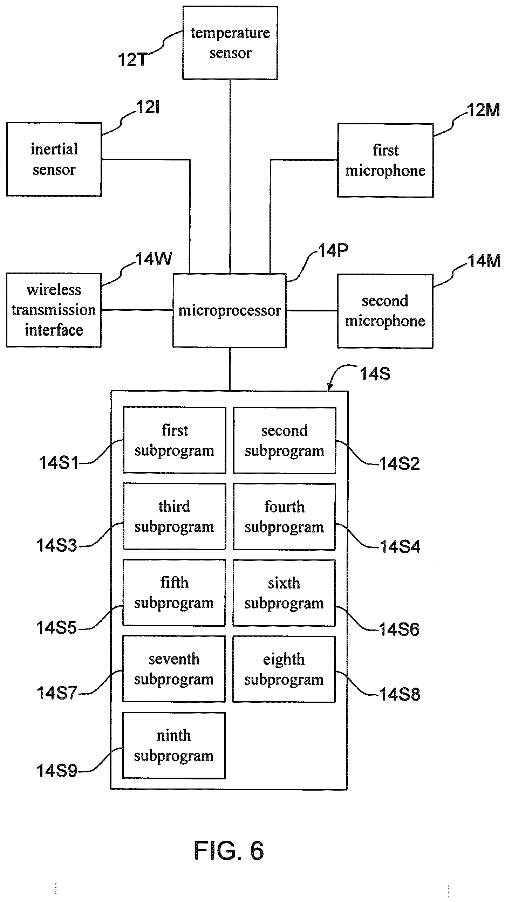

FIG. 6 shows a block diagram of the first microphone 12M, the temperature sensor 12T, the inertial sensor 12I, the second microphone 14M, the microprocessor 14P, the memory 14S, and the wireless transmission interface 14W they are shown in FIG. 5A and FIG. 5B. In one embodiment, the memory 14S stores an application program including instructions, such that in case the application program is executed, the microprocessor 14P is configured for controlling the first microphone 12M, the temperature sensor 12T, the inertial sensor 12I, the second microphone 14M, and the wireless transmission interface 14W, so as to achieve the measurement of physiological parameters (e.g., heart rate and respiratory rate) and the monitoring of the baby's physical conditions. As FIG. 6 shows, the application program consists of a plurality of subprograms, and the plurality of subprograms comprising: a first subprogram 14S1, a second subprogram 14S2, a third subprogram 14S3, a fourth subprogram 14S4, a fifth subprogram 14S5, a sixth 14S6, a seventh subprogram 14S7, an eighth subprogram 14S8, and a ninth subprogram 14S9. It is worth explaining that, not only does the memory 14S can be an embedded flash (eFlash) memory provided in the microprocessor 14P, but the memory 14S can also be a flash memory chip, a hard drive (HD), a solid state drive (SSD), or an USB flash drive that is coupled to the microprocessor 14P.

According to the present invention, the first subprogram 14S1 is compiled to be integrated in the application program by one type of programming language, and includes instructions for configuring the microprocessor 14P to control the temperature sensor 12T to measure a body temperature from the human body (e.g., a body 2 of a baby), thereby generating a body temperature sensing signal. Moreover, the second subprogram 14S2 is compiled to be integrated in the application program by one type of programming language, and includes instructions for configuring the microprocessor 14P to control the first microphone 12M and the second microphone 14M to collect a sound emitted from the human body, thereby generating a first sound signal and a second sound signal, respectively. In addition, the third subprogram 14S3 is compiled to be integrated in the application program by one type of programming language, and includes instructions for configuring the microprocessor 14P to control the inertial sensor 12I to monitor a movement and/or a vibration of the human body, thereby generating a body activity sensing signal. Furthermore, the fourth subprogram 14S4 is compiled to be integrated in the application program by one type of programming language, and includes instructions for configuring the microprocessor 14P to process the body temperature sensing signal, the first sound signal, the second sound signal, and/or the body activity sensing signal. As described in more detail below, the fifth subprogram 14S5 is compiled to be integrated in the application program by one type of programming language, and includes instructions for configuring the microprocessor 14P to apply a signal synchronizing process to the body temperature sensing signal, the first sound signal, the second sound signal, and the body activity sensing signal according to four timestamps that are respectively contained in the body temperature sensing signal, the first sound signal, the second sound signal, and the body activity sensing signal.

As FIG. 6 shows, the sixth 14S6 is compiled to be integrated in the application program by one type of programming language, and includes instructions for configuring the microprocessor 14P to judge whether the human body has at least one physical condition and then determine said physical condition. On the other hand, the seventh subprogram 14S7 is compiled to be integrated in the application program by one type of programming language, and includes instructions for configuring the microprocessor 14P to calculate an estimated body temperature according to the body temperature sensing signal, and to estimate at least one physiological parameter of the human body by processing the first sound signal, the second sound signal and the body activity sensing signal. In one practicable embodiment, the physiological parameter contains heart rate (HR) and/or respiration rate (RR). Moreover, the eighth subprogram 14S8 is compiled to be integrated in the application program by one type of programming language, and includes instructions for configuring the microprocessor 14P to judge whether there is a well contact between the first body 11 and the human body by analyzing the body temperature sensing signal, the body activity sensing signal, a first frequency band and a second frequency band of the first sound signal. According to the particular design of the present invention, before starting to monitor the physical conditions and/or measure the physiological parameters from the baby's body 2, the eighth subprogram 14S8 is executed by the microprocessor 14P, such that the microprocessor 14P is configured to judge whether there is a well contact between the first body 11 and the baby's body 2 or not. After the first body 11 is detected, by the sensor module 1S and the processor module 1P, to have already had a well contact with the baby's body 2, the microprocessor 14P immediately enables the real-time monitoring device 1 to start the measurement of physiological parameters (e.g., heart rate and respiratory rate) and the monitoring of the baby's physical conditions. By such design, the foregoing well-contact detecting function is not only helpful in making the real-time monitoring device 1 to achieve the measurement of physiological parameters and physical conditions of the baby with high accuracy, but also significantly save the power consumption of the real-time monitoring device 1. On the other hand, the ninth subprogram 14S9 is compiled to be integrated in the application program by one type of programming language, and includes instructions for configuring the microprocessor 14P to generate a warning signal in case of there is existing said physical condition and/or at least one said physiological parameter exceeding a normal range, and then to transmit a warning signal to the electronic device 3 through the wireless transmission interface 14W.

As described in more detail below, during the fact that the real-time monitoring device 1 works normally, the microprocessor 14P executes the first subprogram 14S1, such that the temperature sensor 12T is controlled to measure a body temperature from a human body (e.g., a body 2 of a baby), thereby generating a body temperature sensing signal. Simultaneously, the microprocessor 14P executes the second subprogram 14S2, such that the first microphone 12M and the second microphone 14M are controlled to collect a sound emitted from the baby's body 2, thereby generating a first sound signal and a second sound signal, respectively. Moreover, the microprocessor 14P also executes the third subprogram 14S3, such that the inertial sensor 12I is controlled to monitor the movement and/or a vibration of the baby's body 2, thereby generating a body activity sensing signal.

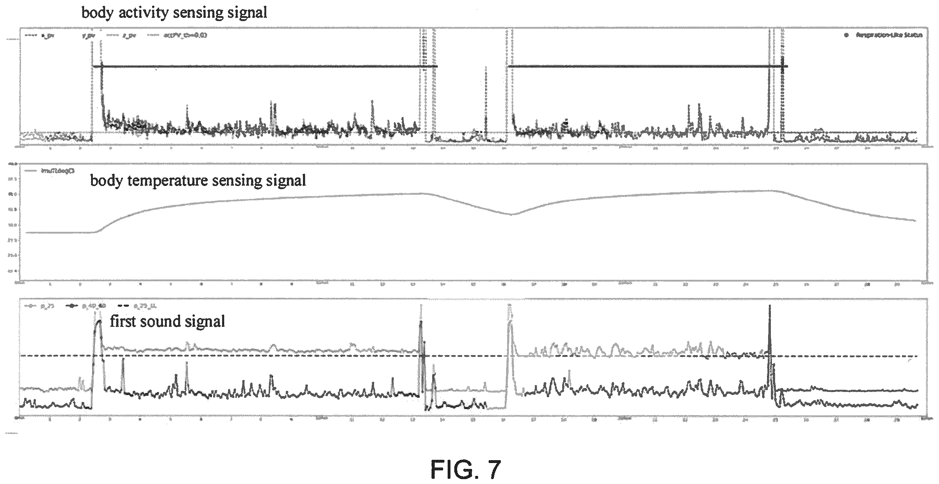

FIG. 7 shows a measured data graph of the body activity sensing signal, the body temperature sensing signal and the first sound signal. In case the eighth subprogram 14S8 is executed, the microprocessor 14P is configured to firstly determine whether the body activity sensing signal includes a signal segment for describing a breath variation of the baby. In other words, there is said signal segment in the body activity sensing signal means that the first body 11 of the sensor module 1S have already had a well contact with the baby's belly. On the other hand, it is also practicable to judge whether there is a well contact between the first body 11 and the baby's belly by analyzing the body temperature sensing signal. For example, in case the first body 11 is set to well contact the baby's belly by a body contacting surface, there is an obviously signal variation occurring in the body temperature sensing signal (as shown in FIG. 7). Of course, if there is a signal variation suddenly occurring in the body temperature sensing signal in case of the real-time monitoring device being operated, it means that the first body 11 is no longer having a well contact with the baby's belly. Particularly, it is also practicable to judge whether there is a well contact between the first body 11 and the baby's belly by analyzing the first sound signal. According to the measured data graph of FIG. 7, when the first body 11 is set to well contact the baby's belly, the magnitude of a first frequency band in the first sound signal shows an abruptly enhancement, wherein the first frequency band of the first sound signal includes the sound emitted from the baby's body 2 falls below the 25 Hz frequency band. Moreover, According to the measured data graph of FIG. 7, when the first body 11 is set to well contact the baby's belly, the magnitude of a second frequency band in the first sound signal also shows an abruptly enhancement, wherein the second frequency band of the first sound signal includes the sound emitted from the baby's body 2 falls between 40 Hz and 60 Hz.

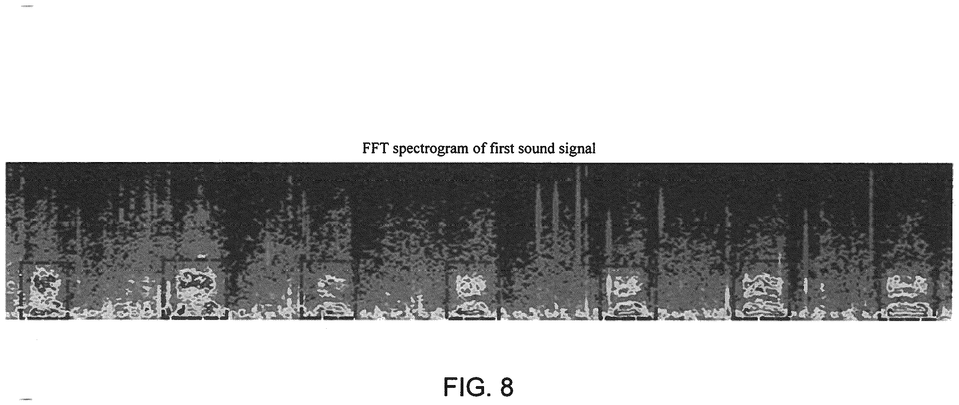

It needs to further explain that, the microprocessor 14P is provided with an analog-to-digital (A/D) convertor therein. After the microprocessor 14P receives the body activity sensing signal, the first sound signal and the second sound signal, the A/D convertor is enabled to directly digitize the first sound signal, digitize the second sound signal using a first sampling rate, and digitize the body activity sensing signal using a second sampling rate. In one embodiment, the first sampling rate is not greater than 4 KHz (i.e., ≦4 KHz), and the second sampling rate is not greater than 120 Hz (i.e., ≦120 Hz). After that, the microprocessor 14P executes the fourth subprogram 14S4, so as to process the first sound signal, the second sound signal, and/or the body activity sensing signal. For example, the microprocessor 14P apply a FFT (fast Fourier transform) process to the first sound signal and the second sound signal, thereby generating a first FFT spectrogram of the first sound signal and a second FFT spectrogram of the second sound signal. FIG. 8 illustrates a FFT spectrogram of the first sound signal containing airway obstruction feature. As FIG. 8 shows, after the first sound signal has received a FFT treatment, it is allowed to find out at least one signal segment containing airway obstruction feature(s) from the FFT spectrogram of the first sound signal.

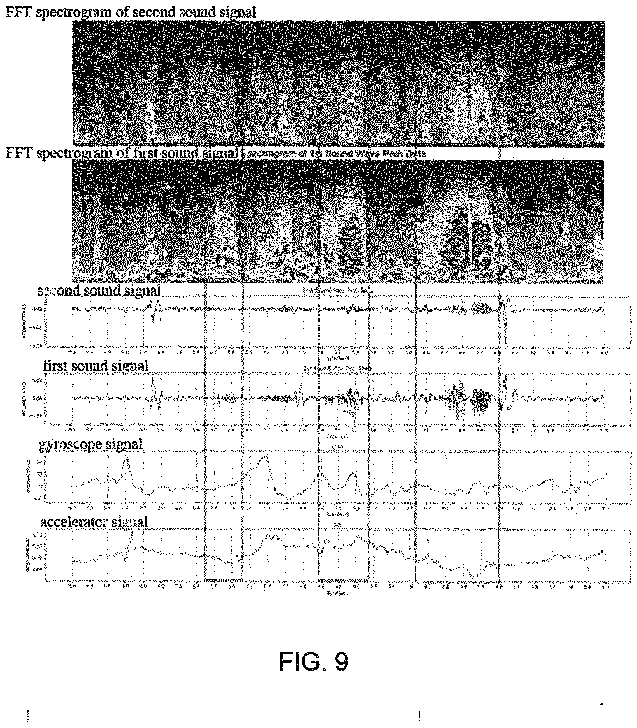

FIG. 9 illustrates a measured data graph of the first sound signal, the FFT spectrogram of the first sound signal, the second sound signal, the FFT spectrogram of the second sound signal, and body activity sensing signal. As FIG. 9 shows, by comparing the first sound signal with the second sound signal, it is able to know that a magnitude variation occurring in the first sound signal (i.e., the sound collected by the first microphone 12M from the baby's belly) is caused by environment noise, or is indeed a reflect of an inner sound of the baby's belly. Therefore, after said magnitude variation of the first sound signal is verified as a reflect of an inner sound of the baby's belly, the microprocessor 14P is subsequently configured to find out at least one signal segment containing abnormal bowel sound feature(s) from the first sound signal, the second sound signal and the body activity sensing signal (including gyroscope signal and accelerator signal). The segment containing abnormal bowel sound features are labeled by gray rectangular frame in FIG. 9.

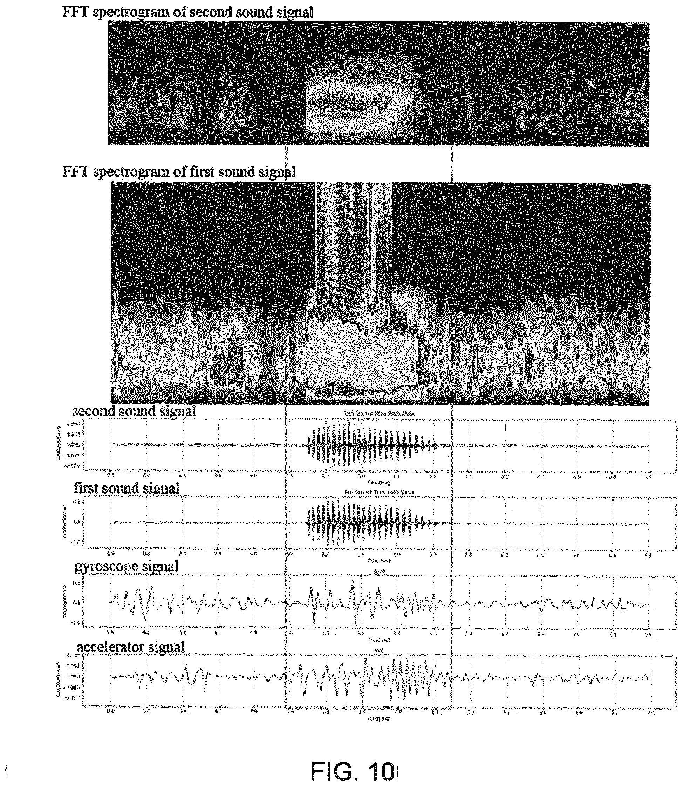

With reference to FIG. 10, there is a measured data graph of the first sound signal, the FFT spectrogram of the first sound signal, the second sound signal, the FFT spectrogram of the second sound signal, and body activity sensing signal provided. As FIG. 10 shows, by comparing the first sound signal with the second sound signal, it is able to know that a magnitude variation occurring in the first sound signal is caused by environment noise, or is indeed a reflect of an inner sound of the baby's belly. Subsequently, after said magnitude variation of the first sound signal is verified as a reflect of an inner sound of the baby's belly, the microprocessor 14P is configured to find out at least one signal segment containing excretion feature(s) from the first sound signal, the second sound signal and the body activity sensing signal (including gyroscope signal and accelerator signal). The segment containing excretion features are labeled by gray rectangular frame in FIG. 10.

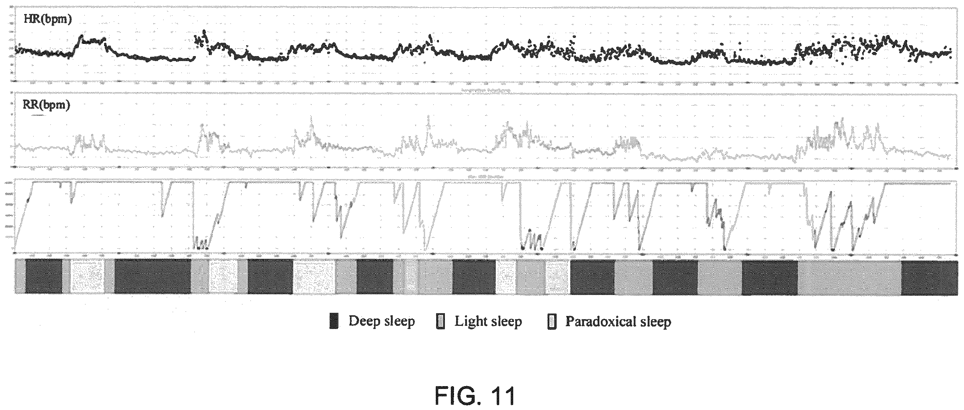

FIG. 11 illustrates a measured data graph of hearth rate signal and respiration rate signal. As FIG. 11 shows, after processing the body activity sensing signal, a hearth rate (HR) signal and respiration rate (RR) signal are therefore obtained. Next, it is able to find out at least one signal segment containing features of going into a deep sleep, going into a light sleep and going into a paradoxical sleep from the HR signal and the RR signal. Moreover, the microprocessor 14P can also estimates physiological parameters of the baby, including heart rate and respiration rate.

Therefore, through above descriptions, all embodiments and their constituting elements of the real-time monitoring device for human body according to the present invention have been introduced completely and clearly.

## Claims

1. A real-time monitoring device for human body, comprising:
a sensor module (1S), comprising a first body (11) having a first accommodation space (11A1) and a first circuit assembly disposed in the first accommodation space (11A1), wherein a first cover (11C1) being connected to a first opening of the first accommodation space (11A1) so as to shield the first circuit assembly, and wherein the first circuit assembly comprises a first microphone (12M), a temperature sensor (12T) and an inertial sensor (121); and
a processor module (1P), comprising a second body (13) and a second circuit assembly disposed in the second body (13), wherein the second circuit assembly comprises a second microphone (14M), a microprocessor (14P), a memory (14S), and a wireless transmission interface (14W);
wherein:
the first body (11) has a body contacting surface for use in contacting a human body, and the body contacting surface is provided with a circular recess (111R) thereon;
the circular recess (111R) has a depth and a diameter in a range between 4.5 mm and 20 mm, such that a ratio of the diameter to the depth being not greater than 6;
an aperture (111O) is formed on a bottom of the first accommodation space (11A1), such that the first microphone (12M) is exposed out of the first body (11) via the aperture (111O); and
the memory (14S) stores an application program including instructions, such that in case the application program is executed, the microprocessor (14P) is configured for:
controlling the temperature sensor (12T) to measure a body temperature from the human body, thereby generating a body temperature sensing signal;
controlling the first microphone (12M) to collect a first sound emitted from the human body, thereby generating a first sound signal;
controlling the inertial sensor (121) to monitor a movement and/or a vibration of the human body, thereby generating a body activity sensing signal;
controlling the second microphone (14M) to collect a second sound emitted from the human body, thereby generating a second sound signal;
judging whether the human body has at least one physical condition by comparing the first sound signal with the second sound signal; and
analyzing the body temperature sensing signal, the first sound signal, the second sound signal, and the body activity sensing signal, so as to determine said physical condition includes at least one selected from a group consisting of excretion, abnormal heart rate (HR), abnormal respiration rate (RR), emission of abnormal bowel sounds, airway obstruction, going into a deep sleep, going into a light sleep, and going into a paradoxical sleep.

2. The real-time monitoring device for human body of claim 1, wherein a minimum value of the depth of the circular recess (111R) is 1.5 mm.

3. The real-time monitoring device for human body of claim 1, wherein the second body (13) has a second accommodation space (13A2) for receiving the second circuit assembly therein, and a second cover (13C2) being connected to a second opening of the second accommodation space (13A2) so as to shield the second circuit assembly.

4. The real-time monitoring device for human body of claim 3, further comprising:
an article supporting unit (14F), being disposed in the second accommodation space (13A2), and consisting of a platform (14F1) and a plurality of supporting rods (14F2); wherein the platform (14F1) is faced to a bottom of the second accommodation space (13A2), and the second circuit assembly is positioned in a space formed by the plurality of supporting rods (14F2) and a bottom surface of the platform (14F1).

5. The real-time monitoring device for human body of claim 4, wherein the processor module (1P) further comprises:
a wireless charging module (1P1), being disposed on a top surface of the platform (14F1), and being coupled to the second circuit assembly; and
a battery (14B), being coupled to the second circuit assembly.

6. The real-time monitoring device for human body of claim 1, wherein a body connecting member (1B) is connected between the first body (11) and the second body (13), and the bendable body connecting member (1B) being provided with an electrical connecting component therein, such that the first circuit assembly is coupled to the second circuit assembly through the electrical connecting component.

7. The real-time monitoring device for human body of claim 6, wherein the second body (13), the body connecting member (1B) and the first body (11) are allowed to be fixed on a mounting kit (1K), such that after disposing the mounting kit (1K) on an article that is worn on the human body, the first body (11) is set to contact the human body by the body contacting surface thereof.

8. The real-time monitoring device for human body of claim 1, wherein the second body (13) and the first body (11) are allowed to be connected with a device fixing member (1PT), such that the second body (13) and the first body (11) are allowed to be attached onto the human body through the device fixing member (1PT), thereby making the first body (11) contact the human body by the body contacting surface thereof.

## Patentansprüche

1. Echtzeit-Überwachungsvorrichtung für den menschlichen Körper, umfassend:
ein Sensormodul (1S), das einen ersten Körper (11) mit einem ersten Aufnahmeraum (11A1) und eine in dem ersten Aufnahmeraum (11A1) angeordnete erste Schaltungsanordnung umfasst, wobei eine erste Abdeckung (11C1) mit einer ersten Öffnung des ersten Aufnahmeraums (11A1) verbunden ist, um die erste Schaltungsanordnung abzuschirmen, und wobei die erste Schaltungsanordnung ein erstes Mikrofon (12M), einen Temperatursensor (12T) und einen Trägheitssensor (12I) umfasst; und
ein Prozessormodul (1P), das einen zweiten Körper (13) und eine in dem zweiten Körper (13) angeordnete zweite Schaltungsanordnung umfasst, wobei die zweite Schaltungsanordnung ein zweites Mikrofon (14M), einen Mikroprozessor (14P), einen Speicher (14S) und eine Drahtlos-Übertragungsschnittstelle (14W) umfasst,
wobei:
der erste Körper (11) eine Körperkontaktfläche zur Verwendung beim Kontaktieren eines menschlichen Körpers aufweist und die Körperkontaktfläche mit einer kreisförmigen Aussparung (111R) darauf versehen ist;
die kreisförmige Aussparung (111R) eine Tiefe und einen Durchmesser in einem Bereich zwischen 4,5 mm und 20 mm aufweist, so dass das Verhältnis von Durchmesser zu Tiefe nicht größer als 6 ist;
eine Öffnung (111O) an einem Boden des ersten Aufnahmeraums (11A1) ausgebildet ist, so dass das erste Mikrofon (12M) durch die Öffnung (111O) aus dem ersten Körper (11) freigelegt ist; und
der Speicher (14S) ein Anwendungsprogramm speichert, das Anweisungen enthält, so dass der Mikroprozessor (14P) im Falle der Ausführung des Anwendungsprogramms konfiguriert ist zum:
Steuern des Temperatursensors (12T), um die Körpertemperatur des menschlichen Körpers zu messen und dadurch ein Körpertemperaturerfassungssignal zu erzeugen;
Steuern des ersten Mikrofons (12M), um einen ersten vom menschlichen Körper abgegebenen Ton zu sammeln und dadurch ein erstes Tonsignal zu erzeugen;
Steuern des Trägheitssensors (12I), um eine Bewegung und/oder eine Vibration des menschlichen Körpers zu überwachen und dadurch ein Körperaktivitätserfassungssignal zu erzeugen;
Steuern des zweiten Mikrofons (14M), um einen zweiten vom menschlichen Körper abgegebenen Ton zu sammeln und dadurch ein zweites Tonsignal zu erzeugen;
Beurteilen, ob der menschliche Körper mindestens einen physischen Zustand aufweist, indem das erste Tonsignal mit dem zweiten Tonsignal verglichen wird; und
Analysieren des Körpertemperaturerfassungssignals, des ersten Tonsignals, des zweiten Tonsignals und des Körperaktivitätserfassungssignals, um zu bestimmen, dass der körperliche Zustand mindestens eines enthält, das aus einer Gruppe ausgewählt ist, bestehend aus Ausscheidung, abnormaler Herzfrequenz (HR), abnormaler Atmungsrate (RR), Emission abnormaler Darmgeräusche, Atemwegsobstruktion, Eintreten in einen tiefen Schlaf, Eintreten in einen leichten Schlaf und Eintreten in einen paradoxen Schlaf

2. Echtzeit-Überwachungsvorrichtung für den menschlichen Körper gemäß Anspruch 1, wobei ein Mindestwert der Tiefe der kreisförmigen Aussparung (111R) 1,5 mm beträgt.

3. Echtzeit-Überwachungsvorrichtung für den menschlichen Körper gemäß Anspruch 1, wobei der zweite Körper (13) einen zweiten Aufnahmeraum (13A2) zur Aufnahme der zweiten Schaltungsanordnung darin und eine zweite Abdeckung (13C2) aufweist, die mit einer zweiten Öffnung des zweiten Aufnahmeraums (13A2) verbunden ist, um die zweite Schaltungsanordnung abzuschirmen.

4. Echtzeit-Überwachungsvorrichtung für den menschlichen Körper gemäß Anspruch 3, ferner umfassend:
eine Artikelträgereinheit (14F), die in dem zweiten Aufnahmeraum (13A2) angeordnet ist und aus einer Plattform (14F1) und einer Vielzahl von Stützstäben (14F2) besteht; wobei die Plattform (14F1) einem Boden des zweiten Aufnahmeraums (13A2) zugewandt ist und die zweite Schaltungsanordnung in einem Raum positioniert ist, der durch die Vielzahl von Stützstäben (14F2) und eine Bodenfläche der Plattform (14F1) gebildet wird.

5. Echtzeit-Überwachungsvorrichtung für den menschlichen Körper gemäß Anspruch 4, wobei das Prozessormodul (1P) ferner umfasst:
ein Drahtlos-Lademodul (1P1), das auf einer oberen Fläche der Plattform (14F1) angeordnet und mit der zweiten Schaltungsanordnung gekoppelt ist; und
eine Batterie (14B), die mit der zweiten Schaltungsanordnung verbunden ist.

6. Echtzeit-Überwachungsvorrichtung für den menschlichen Körper gemäß Anspruch 1, wobei ein Körperverbindungselement (1B) zwischen dem ersten Körper (11) und dem zweiten Körper (13) verbunden ist und das biegsame Körperverbindungselement (1B) mit einer elektrischen Verbindungskomponente darin versehen ist, so dass die erste Schaltungsanordnung mit der zweiten Schaltungsanordnung über die elektrische Verbindungskomponente gekoppelt ist.

7. Echtzeit-Überwachungsvorrichtung für den menschlichen Körper gemäß Anspruch 6, wobei der zweite Körper (13), das Körperverbindungselement (1B) und der erste Körper (11) an einem Montagesatz (1K) befestigt werden können, so dass nach dem Anbringen des Montagesatzes (1K) an einem Artikel, der am menschlichen Körper getragen wird, der erste Körper (11) so festgelegt ist, dass er den menschlichen Körper über seine Körperkontaktfläche berührt.

8. Echtzeit-Überwachungsvorrichtung für den menschlichen Körper gemäß Anspruch 1, wobei der zweite Körper (13) und der erste Körper (11) mit einem Vorrichtungsbefestigungselement (1PT) verbunden werden können, so dass der zweite Körper (13) und der erste Körper (11) durch das Vorrichtungsbefestigungselement (1PT) an dem menschlichen Körper befestigt werden können, wodurch der erste Körper (11) den menschlichen Körper mit seiner Körperkontaktfläche berührt.

## Revendications

1. Un dispositif de surveillance en temps réel pour le corps humain, comprenant :
un module capteur (1S), comprenant un premier corps (11) ayant un premier espace de logement (11A1) et un premier ensemble circuit disposé dans le premier espace de logement (11A1), un premier couvercle (11C1) étant relié à une première ouverture du premier espace de logement (11A1) de manière à protéger le premier ensemble circuit, et le premier ensemble circuit comprenant un premier microphone (12M), un capteur de température (12T) et un capteur inertiel (12I) ; et
un module processeur (1P), comprenant un deuxième corps (13) et un deuxième ensemble circuit disposé dans le deuxième corps (13), le deuxième ensemble circuit comprenant un deuxième microphone (14M), un microprocesseur (14P), une mémoire (14S) et une interface de transmission sans fil (14W) ;
dans lequel :
le premier corps (11) présente une surface de contact avec le corps destinée à venir en contact avec un corps humain, et la surface de contact avec le corps présente un évidement circulaire (111R) sur elle ;
l'évidement circulaire (111R) a une profondeur et un diamètre compris entre 4,5 mm et 20 mm, le rapport entre le diamètre et la profondeur n'étant pas supérieur à 6 ;
une ouverture (111O) est formée sur le fond du premier espace de logement (11A1), de sorte que le premier microphone (12M) est exposé à l'extérieur du premier corps (11) par l'ouverture (111O) ; et
la mémoire (14S) stocke un programme d'application comprenant des instructions, de sorte que dans le cas où le programme d'application est exécuté, le microprocesseur (14P) est configuré pour :
commander le capteur de température (12T) pour mesurer la température corporelle du corps humain, générant ainsi un signal de détection de la température corporelle ;
commander le premier microphone (12M) pour recueillir un premier son émis par le corps humain, générant ainsi un premier signal sonore ;
commander le capteur inertiel (12I) pour surveiller un mouvement et/ou une vibration du corps humain, générant ainsi un signal de détection de l'activité du corps ;
commander le deuxième microphone (14M) pour recueillir un deuxième son émis par le corps humain, générant ainsi un deuxième signal sonore ;
juger si le corps humain présente au moins une condition physique en comparant le premier signal sonore au deuxième signal sonore ; et
analyser le signal de détection de la température corporelle, le premier signal sonore, le deuxième signal sonore et le signal de détection de l'activité corporelle, de manière à déterminer que ladite condition physique comprend au moins un élément sélectionné dans un groupe comprenant l'excrétion, une fréquence cardiaque anormale (FC), une fréquence respiratoire anormale (FR), l'émission de bruits intestinaux anormaux, l'obstruction des voies respiratoires, l'entrée dans un sommeil profond, l'entrée dans un sommeil léger et l'entrée dans un sommeil paradoxal.

2. Le dispositif de surveillance en temps réel du corps humain selon la revendication 1, dans lequel la valeur minimale de la profondeur de l'évidement circulaire (111R) est de 1,5 mm.

3. Le dispositif de surveillance en temps réel du corps humain selon la revendication 1, dans lequel le deuxième corps (13) a un deuxième espace de logement (13A2) pour recevoir le deuxième ensemble circuit à l'intérieur de lui, et un deuxième couvercle (13C2) étant connecté à une deuxième ouverture du deuxième espace de logement (13A2) de manière à protéger le deuxième ensemble circuit.

4. Le dispositif de surveillance en temps réel du corps humain selon la revendication 3, comprenant en outre
une unité (14F) de support d'article, disposée dans le deuxième espace de logement (13A2), et constituée d'une plate-forme (14F1) et d'une pluralité de tiges de support (14F2) ; la plate-forme (14F1) est orientée vers un fond du deuxième espace de logement (13A2), et le deuxième ensemble circuit est positionné dans un espace formé par la pluralité de tiges de support (14F2) et une surface inférieure de la plate-forme (14F1).

5. Le dispositif de surveillance en temps réel du corps humain selon la revendication 4, dans lequel le module processeur (1P) comprend en outre :
un module de chargement sans fil (1P1), disposé sur la surface supérieure de la plate-forme (14F1) et relié au deuxième ensemble circuit ; et
une batterie (14B), reliée au deuxième ensemble circuit.

6. Le dispositif de surveillance en temps réel du corps humain selon la revendication 1, dans lequel un élément (1B) de connexion du corps est connecté entre le premier corps (11) et le deuxième corps (13), et l'élément pliable (1B) de connexion du corps est pourvu en lui d'un composant de connexion électrique, de sorte que le premier ensemble circuit est relié au deuxième ensemble circuit par le biais du composant de connexion électrique.

7. Le dispositif de surveillance en temps réel du corps humain selon la revendication 6, dans lequel le deuxième corps (13), l'élément (1B) de connexion du corps et le premier corps (11) sont aptes à être fixés sur un kit de montage (1K), de sorte qu'après avoir disposé le kit de montage (1K) sur un article qui est porté sur le corps humain, le premier corps (11) est réglé pour entrer en contact avec le corps humain par sa surface de contact avec le corps.

8. Le dispositif de surveillance en temps réel du corps humain selon la revendication 1, dans lequel le deuxième corps (13) et le premier corps (11) sont aptes à être reliés à un élément de fixation de dispositif (1PT), de sorte que le deuxième corps (13) et le premier corps (11) sont aptes à être fixés sur le corps humain par l'intermédiaire de l'élément de fixation de dispositif (1PT), ce qui permet au premier corps (11) d'entrer en contact avec le corps humain par sa surface de contact avec le corps.
